# EUROPEAN PATENT APPLICATION

(11) **EP 2 842 547 A1**
(43) Date of publication of application: **04.03.2015**
(21) Application number: 13181871.8
(22) Date of filing: 27.08.2013
(51) Int. Cl.: A61K 9/16, A61K 9/50, A61K 31/00

(54) **Improved fenofibrate compositions**

(71) Applicant: Freund Pharmatec Ltd., Tullamore Offaly (IE)
(72) Inventor: Ikeda, Masayuki, Co. Offaly (IE); Sivadas, Neeraj, Co. Offaly (IE); Demion, Anthony, Co. Offaly (IE); Gavin, Aoibheann, Co. Offaly (IE); Flaherty, Ciara, Co. Offaly (IE)
(74) Representative: Gates, Marie Christina Esther

(57) **Abstract**

The present invention relates to improved pharmaceutical compositions of fenofibrate, having high bioavailability and to a method for preparing such compositions, improved fenofibrate formulation comprising: fenofibrate, one or more polyglycolized glycerides and gelatin.

## Description

### Field of the Invention

The present invention relates to improved pharmaceutical compositions of fenofibrate, having high bioavailability and to a method for preparing such compositions.

### Background to the Invention

Fenofibrate is a hydrophobic, lipid-regulating agent used in the treatment of adult endogenous hyperlipidaemia, hypercholesterolaemia and hypertriglyceridaemia. The chemical name for fenofibrate is 2-(4-(4-chlorobenzoyl)phenoxy)-2-methyl-propionate and its chemical structure is shown in formula (I):

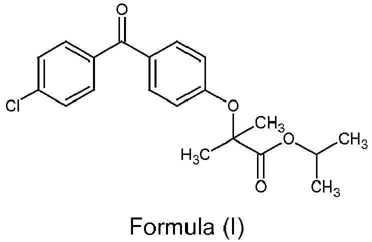

The active metabolite of fenofibrate is its hydrolyzed acid derivative, fenofibric acid. Treatment with fenofibrate leads to reductions in total cholesterol, low-density lipoprotein cholesterol, apolipoprotein B, total triglycerifes and triglyceride rich lipoprotein. Furthermore, an increase in the level of high-density lipoprotein (so called good cholesterol) and apoproteins apoA-I and apoA-II is observed on treatment with fenofibrate.

The action of fenofibrate is mediated through the activation of peroxisome proliferator activated receptor α (PPARα), leading to an increase of lipolysis and subsequent activation of lipoprotein lipase leads to elimination of triglyceride rich particles from plasma and reduced production of apoprotein C-III. The reduction in triglyceride levels leads to modification of low-density lipoprotein cholesterol in terms of size and susceptibility to catabolism.

Fenofibrate is currently marketed as tablets under the trade names Tricor™, Fenoglide^{®} and Triglide^{®} and as capsules under the trade names Antara^{®} and Lipofen^{®}.

Fenofibrate is poorly soluble in water and consequently has limited bioavailability. The drug has poor solubility in gastrointestinal fluid and consequently is poorly absorbed.

Bioavailability is the degree to which an active ingredient, after administration becomes available to the target tissue. Poor bioavailability poses significant problems in the development of pharmaceutical compositions. Active ingredients that are poorly soluble in aqueous media often have insufficient dissolution profiles and consequently have poor bioavailability within an organism after oral administration. In order to circumvent this disadvantage, the administration of multiple therapeutic doses is often necessary.

In recent years, focus in formulation laboratories for improving the bioavailability of hydrophobic pharmacologically active ingredients has been upon reducing particle size. The rate of dissolution of a particulate drug can be increased, by decreasing particle size, through an effective increase in surface area.

Considerable effort has been made to develop methods for controlling drug particle size in pharmaceutical compositions. For example, in order to improve the rate of dissolution of fenofibrate, a wide variety of formulation methods have been employed, including micronization of the active principle, addition of a surfactant and co-micronization of fenofibrate with a surfactant.

US4961890 to Boyer, describes fenofibrate granules, in which fenofibrate is micronized in order to increase its bioavailability. Each fenofibrate granule comprises an inert core, a layer based on fenofibrate and a protective outer layer. The crystalline fenofibrate particles are less than 30 µm in diameter. The binder used is a water soluble polymer, for example polyvinylpyrrolidine, and constitutes an inert water soluble matrix in which the micronized fenofibrate is suspended. The quantity of binder used is such that the amount of fenofibrate released after stirring in a galenical preparation for 1 hour is at least 65%. No examples quantifying specifically, the release of fenofibrate in aqueous media are provided.

WO0016749 discloses a method for preparing novel galenic formulations of fenofibrate with improved bioavailability after oral administration consisting of (a) micronizing fenofibrate; (b) granulating the fenofibrate in the presence of a liquid medium comprising a surfactant, water and water-miscible alcohol; and (c) drying the resulting granular material.

In WO2004028506, pharmaceutical compositions of fenofibrate with high bioavailability after oral administration are disclosed. The immediate release fenofibrate composition comprises an inert hydro-insoluble carrier with at least one layer containing micronized fenofibrate, a hydrophilic polymer and a surfactant; and optionally one or several outer phases or layers. One disclosed example: a composition comprising micronized fenofibrate, microcrystalline cellulose, polyvinyl pyrrolidine, sodium lauryl sulphate, cross-linked polyvinyl pyrrolidine, colloidal silicon dioxide and sodium stearyl fumarate; displayed 53% drug release after 10 minutes in 1 L water using the rotating blade method at 50 rpm according to European Pharmacopoeia, and 91 % drug release after 45 minutes.

In US6277405, an immediate release fenofibrate composition which comprises an inert hydrosoluble carrier covered with at least one layer of micronized fenofibrate with a size less than 20 µm, a hydrophilic polymer and optionally a surfactant and one or several outer phases is described. The claimed compositions have a fenofibrate dissolution of at least 75% after 30 minutes, as measured using the rotating blade method at 75 rpm according to the European Pharmacopoeia, in an aqueous solution of 0.025M sodium lauryl sulphate.

Curtet et al. in US4895726 proposes improving the bioavailability of fenofibrate by co-micronizing it with a solid surfactant such as sodium lauryl-sulfate, wherein the mean particle size of said co-micronized mixture is less than 15 µm. The co-micronizate is then granulated by wet granulation in order to improve the flow capacities of the powder and to facilitate the transformation into gelatin capsules. It was found that the time taken for 50% of fenofibrate to dissolve (i.e. the T 50%) was very significantly reduced when the fenofibrate and the sodium lauryl-sulfate are co-micronized, compared with a mixture of separately micronized fenofibrate and sodium lauryl-sulfate and compared with fenofibrate alone.

In US5882680, β-carotene is added to a middle chain length fatty acid (MCT); the resulting mixture is emulsified to produce a dispersion, which is subsequently homogenised to form a suspension. The suspension then enters a device for the manufacture of seamless capsules, namely a "Spherex" device (manufactured by Freund Industrial Co., Ltd.), the suspension is heated to 35 °C and forms an encapsulating liquid which is subsequently coated by an outer shell forming liquid at 70 °C composed of an aqueous solution of gelatin and sorbitol. The seamless capsule is formed when the encapsulating liquid is passed through the inner tube of series of coaxial tubes and simultaneously the outer shell liquid is passed through the outer tube of the coaxial tubing arrangement, the resulting droplets that form enter a hardening liquid of MCT cooled to 9 °C.

Notwithstanding the state of the art there remains a need for alternative fenofibrate formulations that exhibit improved bioavailability properties.

### OBJECT OF THE INVENTION

It is an object of the invention to provide an improved immediate release fenofibrate formulation. Accordingly, a further object is to provide a novel composition, comprising fenofibrate which has enhanced dissolution and absorption profiles. A particular object is to provide a fenofibrate formulation which has up to 100% release of the active. A still further object is to provide a method for manufacturing the improved fenofibrate composition.

Further advantages of the method of manufacturing the improved fenofibrate composition include the uniformity of drug dispersion in the composition and the ease of manufacture.

### SUMMARY OF THE INVENTION

According to the present invention, there is provided an improved fenofibrate formulation comprising: fenofibrate, one or more polyglycolized glycerides and gelatin. The formulation may be an immediate release formulation.

Another aspect of the present invention provides seamless spheres comprising: fenofibrate, one or more polyglycolized glycerides and gelatin.

The polyglycolized glyceride or mixture thereof may be selected from the group consisting of octanoyl-, nonanoyl-, decanoyl-, stearoyl-, lauroyl-, oleoyl-, lineoyl-, myristoyl-, palmitoyl-, caprylocaproyl- macrogol glycerides. Suitable polyglycolized glycerides include Labrasol®, gelucire® 44/14 and gelucire® 50/13. Particularly preferred is gelucire® 44/14.

The polyglycolized glycerides are mixtures of mono-, di- and tri-glycerides and polyethylene glycol mono- and di-esters obtained either by partial alcoholysis of hydrogenated vegetable oils using polyethylene glycol of relative molecular weight ranging from 200-2000, or by esterification of saturated fatty acids using polyethylene glycol of relative molecular weight ranging from 200-2000 and glycerol (cited in French Pharmacopeia 10th Edition, and recited in WO 95/07696). Gelucire (commercially available from Gattefosse s.a., Saint Priest, France) is sold in the market as a typical good of the saturated polyglycolized glyceride. Examples of Gelucire include Gelucire 35/10, Gelucire 44/14, Gelucire 46/07, Gelucire 50/13, Gelucire 53/10 and the like, each of which is characterized by melting point and HLB (hydrophilic-lipophilic balance). Each Gelucire is designated by two numbers separated by a slash, the first number(two-digit number) indicating its melting point and the second, the HLB (hydrophilic-lipophilic balance). For instance, in Gelucire 44/14, the melting point thereof is 44 °C, and the HLB (hydrophilic-lipophilic balance) thereof is 14.

Preferably, the polyglycolized glycerides of the present invention have a fatty acid composition comprising caprylic acid (C8), capric acid (C10), lauric acid (C12), myristic acid (C14), palmitic acid (C16), stearic acid (C18). More preferably, one or more polyglycolized glycerides of the present invention has a fatty acid composition comprising substantially lauric acid. Even more preferably, the fatty acid composition comprises substantially lauric acid in an amount of from about 30 %w/w to about 60 %w/w based on the total weight of the one or more polyglycolized glycerides.

In one embodiment of the present invention, the ratio of fenofibrate to the one or more polyglycolized glycerides is from about 1:1.5 to about 1:3.5.Preferably the ratio is 1:2.

The spheres or cores may typically have a diameter in the range of 0.5 mm to 7.0 mm, preferably 1.0 mm to 2.5 mm, more preferably, 1.4 mm to 1.7 mm.

Suitably, the pharmaceutical composition of the present invention comprises fenofibrate in an amount of from about 1 %w/w to about 15 %w/w based on the total weight of composition.

Preferably, the pharmaceutical composition of the present invention comprises one or more polyglycolized glycerides in an amount of from about 2 %w/w to about 30 %w/w based on the total weight of composition.

In one embodiment of the present invention the fenofibrate is present in an amount of about 8 %w/w to about 10 %w/w based on the total weight of composition.

In another embodiment of the present invention the one or more polyglycolized glycerides is selected from the group of octanoyl-, nonanoyl-, decanoyl-, stearoyl-, lauroyl-, oleoyl-, lineoyl-, myristoyl-, palmitoyl-, and caprylocaproyl- macrogol glycerides and is present in an amount of about 16 %w/w to about 20 %w/w based on the total weight of composition. In a preferred embodiment the one or more polyglycolized glycerides is gelucire 44/14 and is present in an amount of about 16%w/w to about 20%w/w based on the total weight of composition.

In one embodiment of the present invention the one or more polyglycolized glycerides is present in an amount of from about 16 % w/w to about 20%w/w based on the total weight of composition.

One embodiment of the present invention, provides seamless spheres comprising: fenofibrate, one or more polyglycolized glycerides and gelatin, wherein, the ratio of fenofibrate to one or more polyglycolized glycerides is 1:2; the fenofibrate is present in an amount of about 8 %w/w to about 10 % w/w and the one or more polyglycolized glycerides is present in an amount of about 16% w/w to about 20 %w/w, based on the total weight of composition; and the seamless spheres are from about 1.4 mm to about 1.7 mm in diameter.

One preferred embodiment of the present invention, provides seamless spheres comprising: fenofibrate, gelucire 44/14 and gelatin, wherein, the ratio of fenofibrate to gelucire 44/14 is 1:2; the fenofibrate is present in an amount of about 8 %w/w to about 10 % w/w and the gelucire 44/14 is present in an amount of about 16% w/w to about 20 %w/w, based on the total weight of composition; and the seamless spheres are of from about 1.4 mm to about 1.7 mm in diameter.

In one embodiment the composition comprises: from about 8 to about 10 %w/w fenofibrate; from about 16 to about 20 %w/w of one or more polyglycolized glyceride and from about 70 to about 76 %w/w gelatin based on the total weight of composition.

In another aspect the invention provides a method of manufacturing the pharmaceutical composition of the present invention comprising the steps of:
(i) heating a mixture of fenofibrate and one or more polyglycolized glycerides to a temperature of from about 70 to about 100 °C;
(ii) adding the solution produced in step (i) to a solution of gelatin at a temperature of from about 70 to about 100 °C, and homogenising the two solutions together; and
(iii) processing the solution produced in step (ii) to produce seamless fenofibrate beads or spheres of from about 0.5 mm to about 3.0 mm in diameter.

Steps (i) and (ii) above may be carried out at a temperature of about 85 °C. The one or more polyglycolized glycerides may be selected from the group consisting of gelucire 44/14, Labrasol® and gelucire 50/13. The weight ratio of fenofibrate to one or more polyglycolized glycerides may be from about 1:1.5 to about 1:3.5.

The solution produced in step (ii) may be processed to produce seamless beads or spheres of from about 1.4 mm to about 1.7 mm in diameter.

The invention also provides a pharmaceutical composition comprising seamless beads or spheres of from about 1.4 mm to about 1.7 mm in diameter, made by:
(i) Heating a mixture of fenofibrate and gelucire 44/14 to a temperature from about 70 to about 100 °C;
(ii) Adding the solution produced in step (i) to a solution of gelatin at a temperature of from about 70 to about 100 °C and homogenising the two solutions together; and
(iii) Processing the solution produced in step (ii) using a Spherex™ technology seamless spherical microcapsule manufacturing device, to produce seamless spherical fenofibrate microcapsules.

Another aspect of the present invention provides seamless spheres comprising: fenofibrate, one or more polyglycolized glyceride and gelatin.

A further aspect of the present invention provides seamless spheres comprising: fenofibrate, one or more polyglycolized glycerides and gelatin, wherein the seamless spheres are of from about 0.5 mm to about 3.0 mm in diameter, preferably of from about 1.4 mm to about 1.7 mm in diameter.

In one embodiment, the present provides an improved immediate release seamless sphere fenofibrate formulation comprising: fenofibrate, one or more polyglycolized glyceride and gelatin, wherein the ratio of fenofibrate to one or more polyglycolized glyceride is 1:2 and the seamless spheres are of from about 1.4 mm to about 1.7 mm in diameter.

Suitably, the composition of the present invention comprises gelatin with a bloom strength of from about 80 to about 350. Preferably the bloom strength is from about 180 to 300. The gelatin may be porcine or bovine gelatin.

In one embodiment the gelatin is porcine derived gelatin with a bloom strength of 180 or 300.

In another embodiment the gelatin is bovine derived gelatin with a bloom strength of 225.

One aspect of the present invention involves the manufacture of spheres comprising fenofibrate, one or more polyglycolized glycerides and gelatin, using the process described in EP2586429.

Yet a further aspect provides a method for the manufacture of seamless spheres of the fenofibrate composition of the present invention, comprising the steps of:
(i) Heating a mixture of fenofibrate and one or more polyglycolized glycerides to a temperature of from about 70 °C to about 100 °C;
(ii) Adding resulting solution (i) to a solution of gelatin at a temperature of from about 70 °C to about 100 °C under homogenisation conditions; and
(iii) Processing the resulting homogenised solution (iii) using a Spherex™ microcapsule manufacturing device, to provide seamless spheres of from about 0.5 mm to about 3.0 mm in diameter.

Yet a further aspect provides a method for the manufacture of seamless spheres of the fenofibrate composition of the present invention, comprising the steps of:
(i) Heating a mixture of fenofibrate and gelucire 44/14 to a temperature of from about 70 °C to about 100 °C;
(ii) Adding resulting solution (i) to a solution of gelatin at a temperature of from about 70 °C to about 100 °C under homogenisation conditions; and
(iii) Processing the resulting homogenised solution (iii) using a Spherex™ microcapsule manufacturing device, to provide seamless spheres of from about 0.5 mm to about 3.0 mm in diameter.

Also provided for is a composition according to the present invention for use as a medicament.

Advantageously, the method of manufacture is a single pot process wherein fenofibrate, polyglycolized glyceride and gelatine are mixed together and processed into spheres. The method of manufacture is significantly simpler than that of alternative fenofibrate products. For example the method of manufacture of Antara®, Tricor® and Fenoglide® involve multiple steps and complex processing methods, primarily a pretreatment of fenofibrate (e.g. size reduction) before a final dosage form is produced.

Also provided is a composition according to the present invention for use in the treatment of hyperlipidaemia or mixed dyslipidaemia, hypercholesterolaemia and hypertriglyceridaemia.

The present invention provides an improved immediate release fenofibrate formulation, which has enhanced dissolution and absorption profiles. Advantages include reduced dose dumping, less variability in absorption compared to existing formulations, and much faster release and dissolution due to increased surface area of the spheres. Furthermore, the present invention does not require the addition of disintegrants to achieve this enhanced dissolution profile.

**Brief Description of the Drawings**

Embodiments of the invention will be described, by way of example only, with reference to the accompanying drawings in which:

**Figure 1** shows the dissolution profiles for fenofibrate formulations wherein the solubiliser is varied;

**Figure 2** shows the dissolution profiles for fenofibrate formulations of the present invention which were processed at either 60 °C or 85 °C.

**Figure 3** shows the dissolution profiles for fenofibrate formulations with varying drug loadings and drug:solubiliser ratios.

### Detailed Description of the Invention

The gelatin spheres of the present invention, incorporating fenofibrate, were manufactured according to the teachings of Freund Industrial Co. Ltd. EP2586429 the teachings of which are incorporated herein by reference. This technology is based on the principle that a laminar liquid jet can be broken into equally sized droplets by a superimposed vibration. When the droplets come in contact with a hardening liquid, they undergo gelation leading to the formation of spheres. This technique enables high-speed production of uniformly sized spheres.

Gelucires are polyethylene glycol (PEG) glycerides (or polyglycolized glycerides) composed of mono-, di- and triglycerides and mono- and diesters of PEG. They are inert semi-solid waxy amphiphilic excipients with surface-active properties that spontaneously form a fine dispersion or emulsion upon contact with water.

Examples of gelucires® include gelucire® 44/14, Labrasol® and gelucire® 50/13.

Gelatin is obtained by the partial hydrolysis of collagenous material, such as skin, connective tissues, or animal bones. There are two main classes of gelatin, Type A gelatin, which is obtained from acid-processing of porcine skins and exhibits an isoelectric point between pH 7 and pH 9; and Type B gelatin which is obtained from the alkaline-processing of bovine bone and skin and exhibits an isoelectric point between pH 4.7 and pH 5.2. It will be appreciated by those skilled in the art that varying blends of gelatin are available with varying bloom strength characteristics.

In the examples disclosed below, porcine and bovine derived gelatin are used, however, the skilled person will be appreciate that other sources of gelatin are equally suitable.

In the examples outlined below, Tricor® was used as a comparative sample. Tricor® is a commercially available formulation of fenofibrate and Tricor® 48 mg tablets were employed in comparative experiments.

**Example 1: Selection of Solubiliser**

**Manufacturing Procedure:** Fenofibrate and solubiliser were melted together at 85 °C. (Note the melting point of Fenofibrate is 79-81 °C). A clear solution was obtained. Gelatin was dispersed in water, allowed to swell and melted at 85 °C. The drug/solubiliser solution was added to the gelatin solution (20 %w/w concentration) under homogenisation. The resulting mixture was used to form spherical beads of size 1.4-1.7 mm using Spherex^{™} technology.

**Dissolution Profile:** The drug release profiles of, the formulations shown in Table 1 (vide infra), and a reference product (Tricor^{®} 48 mg tablets) were tested using a dissolution medium consisting of 0.72% Sodium lauryl sulphate in water. USP Apparatus II (Paddle) was used.

Conditions: Volume of media (900 mL); Media temperature (37 °C); Paddle rotation speed (50 rpm); Samples were taken after 10, 20, 30, 45 and 60 minutes.

**Table 1: Fenofibrate formulations with varying solubilisers.**

| | %w/wwt.% | | | | |
|---|---|---|---|---|---|
| **Formulation No.** | **EXPROD-0228A** | **EXPROD-0228B** | **EXPROD-0230A** | **EXPROD-0230B** | **EXPROD-0230C** |
| Fenofibrate | 9.54 | 9.54 | 9.54 | 9.54 | 9.70 |
| Kolliphor HS 15 | 19.04 | 0 | 0 | 0 | 0 |
| Maisine 35-1 | 0 | 0 | 0 | 19.04 | 0 |
| Pecol | 0 | 19.04 | 0 | 0 | 0 |
| Gelucire 44/14 | 0 | 0 | 19.04 | 0 | 0 |
| Gelatin | 71.42 | 71.42 | 71.42 | 71.42 | 90.3 |
| Note: all values refer to percentage dry weight composition of the final formulation. | | | | | |

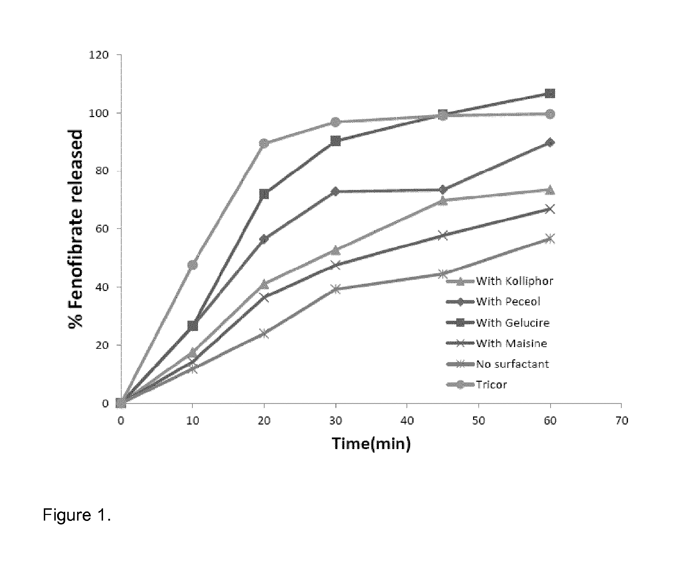

In Figure 1 the effect of varying the solubiliser on the dissolution profile of each fenofibrate formulation is shown. Formulation EXPROD-0230A in which gelucire 44/14 (i.e. lauroyl polyglycolized glyceride) was used as the solubiliser proved the to be the most effective, with complete release of Fenofibrate being achieved after 60 minutes.

**Example 2**

**Effect of processing temperature on drug release**

It was also found that maintaining the processing temperature above the melting point of fenofibrate is critical in achieving the desired release profile. Formulations containing Gelucire 44/14 were manufactured as described above at 60 °C and 85 °C (Table 2) and the release profiles were tested using the conditions described vide supra.

**Table 2**

| | **%w/w** | |
|---|---|---|
| Formulation No. and processing temperature | **EXPROD-0232A @ 60 °C** | **EXPROD-0234A** @ 85 °C |
| Fenofibrate | 9.54 | 9.54 |
| Gelucire 44/14 | 19.04 | 19.04 |
| Gelatin | 71.4 | 71.4 |
| Note: all values refer to percentage dry weight composition of the final formulation. | | |

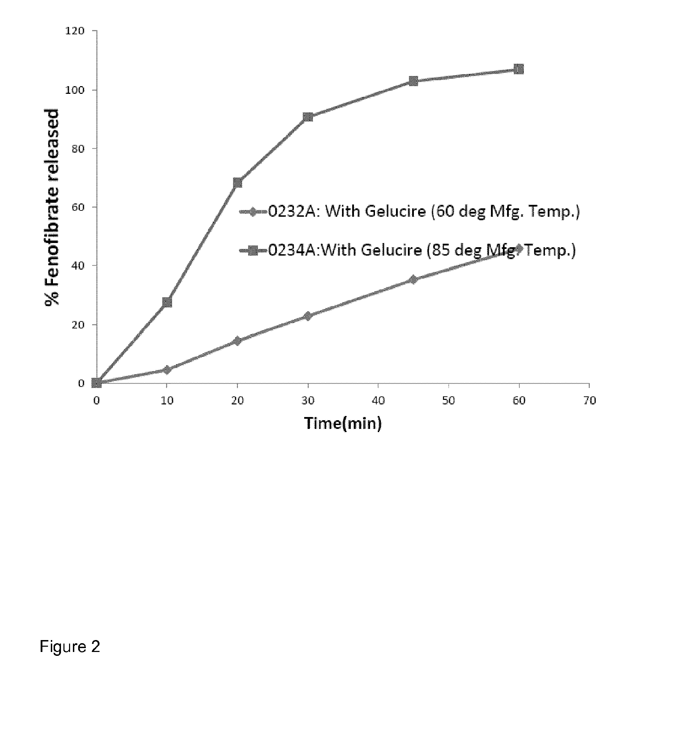

In Figure 2 the effect of different processing temperatures on the dissolution profile of formulations EXPROD-0232A and EXPROD-0234B (which have the same composition) is shown. Figure 2 shows the marked effect that the processing temperature has on the dissolution profile for a given composition.

Figure 2 illustrates the importance of carrying out the manufacturing process at 85 °C. When the manufacturing process is carried out at lower temperature, the dissolution rate of the resulting formulation is significantly reduced.

**Example 3 : Effect of drug loading and drug:solubiliser ratio on drug**

### release

The effects of drug loading and the ratio of fenofibrate to solubiliser were also investigated.

Formulations with varying drug loading and fenofibrate:gelucire 44/14 ratios as shown in Table 3 (vide infra), were prepared using the manufacturing methods described above at a processing temperature of 85 °C.

**Table 3**

| | %w/w | | |
|---|---|---|---|
| **Formulation No.** | **EXPROD-0234A** | **EXPROD-0236A** | **EXPROD-0236B** |
| Fenofibrate | 9.54 | 14.86 | 15.01 |
| Gelucire 44/14 | 19.04 | 29.73 | 15.01 |
| Gelatin | 71.4 | 55.4 | 69.98 |
| Note: all values refer to percentage dry weight composition of the final formulation | | | |

The drug release profiles of the formulations in Table 3 were tested using the conditions described above.

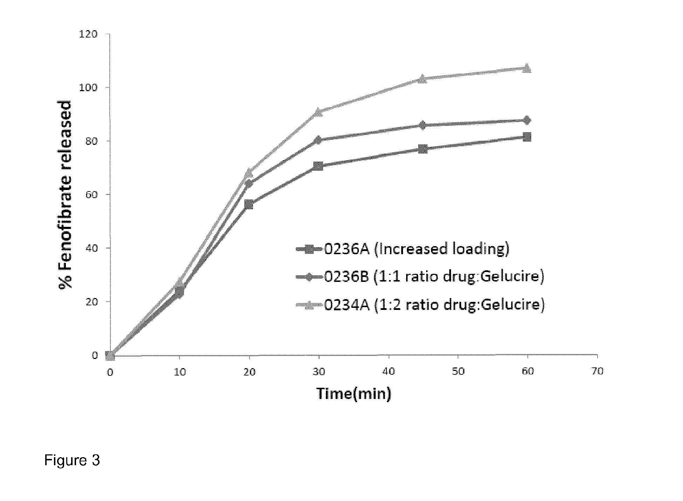

Figure 3 shows the dissolution profiles of formulations with varied drug loadings and the drug:solubiliser ratios. Drug release from the formulation with increased fenofibrate loading i.e. EXPROD-0236A was lower than that for EXPROD-0234A, despite the fact that the ratio of fenofibrate to gelucire 44/14 was the same in each formulation. This demonstrates that the amount of gelatin in the formulation (71.4% versus 55.4%) is also a factor in obtaining the desired drug release. Similarly, a higher drug release was obtained with a 1:2 ratio of drug:solubiliser than with a 1:1 ratio.

The words "comprises/comprising" and the words "having/including" when used herein with reference to the present invention are used to specify the presence of stated features, integers, steps or components but do not preclude the presence or addition of one or more other features, integers, steps, components or groups thereof.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable sub-combination.

## Claims

1. A pharmaceutical composition comprising:
(a) fenofibrate;
(b) one or more polyglycolized glycerides; and
(c) gelatin.

2. A pharmaceutical composition according to claim 1 wherein the one or more polyglycolized glycerides is selected from the group of octanoyl-, nonanoyl-, decanoyl-, stearoyl-, lauroyl-, oleoyl-, lineoyl-, myristoyl-, palmitoyl-, and caprylocaproyl- macrogol glycerides.

3. A pharmaceutical composition according to claim 1 or 2 wherein the fenofibrate is present in an amount of from about 1 to about 15 %w/w based on the total weight of the composition.

4. A pharmaceutical composition according to any preceding claim wherein the one or more polyglycolized glycerides is present in an amount of from about 2 to about 30 %w/w, based on the total weight of the composition.

5. A pharmaceutical composition according to any preceding claim, wherein the one or more polyglycolized glycerides is selected from the group consisting of gelucire 44/14, Labrasol and gelucire 50/13.

6. A pharmaceutical composition according to any preceding claim, wherein the fenofibrate is present in an amount of from about 8 to about 10 %w/w based on the total weight of the composition, and/or the one or more polyglycolized glyceride is present in an amount of from about 16 to about 20 % w/w based on the total weight of the composition.

7. A pharmaceutical composition according to any preceding claim wherein the weight ratio of fenofibrate to one or more polyglycolized glycerides is from about 1:1.5 to about 1:3.5.

8. A pharmaceutical composition according to any preceding claim wherein the weight ratio of fenofibrate to one or more polyglycolized glyceride is 1:2.

9. A pharmaceutical composition according to any preceding claim comprising:
from about 8 to about 10 %w/w fenofibrate; from about 16 to about 20 %w/w one or
more polyoxyglycerides and from about 70 to about 76 %w/w gelatin based on the total weight of composition.

10. A composition as claimed in any preceding claim formulated as seamless beads of from about 0.5 mm to about 7.0 mm in diameter.

11. A method of manufacturing the pharmaceutical composition of the present invention comprising the steps of:
(i) heating a mixture of fenofibrate and one or more polyglycolized glycerides to a temperature of from about 70 to about 100 °C;
(ii) adding the solution produced in step (i) to a solution of gelatin at a temperature of from about 70 to about 100 °C, and homogenising the two solutions together; and
(iii) processing the solution produced in step (ii) to produce seamless fenofibrate beads of from about 0.5 mm to about 3.0 mm in diameter.

12. A method as claimed in claim 11 wherein steps (i) and (ii) are carried out at a temperature of about 85 °C.

13. A method according to any one of claims 11 or 12, wherein the one or more polyglycolized glyceride is selected from the group consisting of gelucire 44/14, Labrasol and gelucire 50/13.

14. A method according to any one of claims 11 to 13 wherein the weight ratio of fenofibrate to one or more polyglycolized glyceride is from about 1:1.5 to about 1:3.5.

15. A method according to any one of claims 11 to 14 wherein the solution produced in step (ii) is processed to produce seamless beads or spheres of from about 1.4 mm to about 1.7 mm in diameter.

16. A pharmaceutical composition comprising seamless beads or spheres of from about 1.4 mm to about 1.7 mm in diameter, made by:
(i) Heating a mixture of fenofibrate and gelucire 44/14 to a temperature from about 70 to about 100 °C;
(ii) Adding the solution produced in step (i) to a solution of gelatin at a temperature of from about 70 to about 100 °C and homogenising the two solutions together; and
(iii) Processing the solution produced in step (ii) using a Spherex™ technology seamless spherical microcapsule manufacturing device, to produce seamless spherical fenofibrate microcapsules.
